# EUROPEAN PATENT SPECIFICATION

(11) **EP 4 640 138 B1**
(45) Date of publication and mention of the grant of the patent: **15.07.2026**
(21) Application number: 23907509.6
(22) Date of filing: 05.12.2023
(51) Int. Cl.: A61B 5/00, G16H 50/20, G16H 50/30, G16H 10/20, G06N 3/09, A61B 5/16

(54) **APPARATUS AND METHOD FOR DIAGNOSING ALZHEIMER'S DISEASE**
VORRICHTUNG UND VERFAHREN ZUR DIAGNOSE VON MORBUS ALZHEIMER
APPAREIL ET MÉTHODE DE DIAGNOSTIC DE LA MALADIE D'ALZHEIMER

(30) Priority: 22.12.2022 KR 20220181389; 23.10.2023 KR 20230141766
(43) Date of publication of application: 29.10.2025
(73) Proprietor: Emocog Inc., Seoul 08708 (KR)
(72) Inventor: BAE, Min Ju, Seoul 08812 (KR); LEE, Da Som, Seoul 06735 (KR)
(74) Representative: Zacco Sweden AB
(86) International application number: PCT/KR2023/019872
(87) International publication number: WO 2024/136223

(56) References cited:
- JP-A- 2022 106 064
- JP-A- 2022 528 790
- KR-A- 20120 070 668
- KR-A- 20220 007 537
- US-A1- 2021 233 660
- US-A1- 2021 233 660
- US-A1- 2021 353 218
- PENG ZIGAO ET AL: "Connected Multi-speech Task for Detecting Alzheimer's Disease Using a Two-Layer Model", 2022 6TH INTERNATIONAL SYMPOSIUM ON COMPUTER SCIENCE AND INTELLIGENT CONTROL (ISCSIC), IEEE, 11 November 2022 (2022-11-11), pages 83 - 88, XP034307983, DOI: 10.1109/ISCSIC57216.2022.00028
- ILIAS LOUKAS ET AL: "Multimodal Deep Learning Models for Detecting Dementia From Speech and Transcripts", FRONTIERS IN AGING NEUROSCIENCE, vol. 14, 17 March 2022 (2022-03-17), CH, XP093317535, ISSN: 1663-4365, DOI: 10.3389/fnagi.2022.830943
- NISHIKAWA KAZU ET AL: "Detecting System Alzheimer's Dementia by 1d CNN-LSTM in Japanese Speech", 2021 IEEE INTERNATIONAL CONFERENCE ON CONSUMER ELECTRONICS (ICCE), IEEE, 10 January 2021 (2021-01-10), pages 1 - 3, XP033916635, DOI: 10.1109/ICCE50685.2021.9427692
- YING YANGWEI ET AL: "Multimodal fusion for alzheimer's disease recognition", vol. 53, no. 12, 1 December 2022 (2022-12-01), NL, pages 16029 - 16040, XP093317328, ISSN: 0924-669X, Retrieved from the Internet <URL:https://link.springer.com/article/10.1007/s10489-022-04255-z/fulltext.html> DOI: 10.1007/s10489-022-04255-z
- BAE MINJU ET AL: "The efficacy of memory load on speech-based detection of Alzheimer's disease", FRONTIERS IN AGING NEUROSCIENCE, vol. 15, 2 June 2023 (2023-06-02), CH, XP093317347, ISSN: 1663-4365, DOI: 10.3389/fnagi.2023.1186786

## Description

### Technical Field

The present disclosure relates to an apparatus and method for diagnosing Alzheimer's disease based on an analysis of speech data of a speaker.

### Background Art

Dementia may refer to a neuropathological state characterized by a deterioration of functions such as cognitive ability, memory, thinking ability, or judgment, due to a gradual decline in brain function. Dementia mainly occurs during the aging process and, as it progresses, may seriously affect social, occupational, and daily life functions.

Dementia has various forms and causes, with a representative form being Alzheimer's disease. Alzheimer's disease is a chronic brain disease caused by the impairment of the function and connectivity of neurons (nerve cells) resulting from damage to brain tissue. The symptoms of dementia may begin with mild memory loss, confusion, or the like, and may gradually worsen as the linguistic ability, judgment, reasoning ability, performance of daily activities, and the like deteriorate.

The following publications are related to speech analysis: PENG ZIGAO ET AL: "Connected Multi-speech Task for Detecting Alzheimer's Disease Using a Two-Layer Model", 2022 6TH INTERNATIONAL SYMPOSIUM ON COMPUTER SCIENCE AND INTELLIGENT CONTROL (ISCSIC), IEEE, 11 November 2022 (2022-11-11), pages 83-88, DOI: 10.1109/ ISCSIC57216.2022.00028, ILIAS LOUKAS ET AL: "Multimodal Deep Learning Models for Detecting Dementia From Speech and Transcripts", FRONTIERS IN AGING NEUROSCIENCE, vol. 14, 17 March 2022 (2022-03-17), CH, ISSN: 1663-4365, DOI: 10.3389/fnagi.2022.830943 and NISHIKAWA KAZU ET AL: "Detecting System Alzheimer's Dementia by 1d CNN-LSTM in Japanese Speech", 2021 IEEE INTERNATIONAL CONFERENCE ON CONSUMER ELECTRONICS (ICCE), IEEE, 10 January 2021 (2021-01-10), pages 1-3, DOI: 10.1109/ICCE50685.2021.9427692.

### Disclosure of Invention

### Technical Problem

An objective of the present disclosure is to improve the accuracy of diagnosing Alzheimer's disease, by analyzing a speaker's speech characteristics collected through a speech task.

An objective of the present disclosure is to accurately predict a cognitive function assessment score by analyzing a speaker's speech characteristics collected through a speech task.

Objectives of the present disclosure are not limited to the foregoing, and other unmentioned objectives or advantages of the present disclosure would be understood from the following description and be more clearly understood from the embodiments of the present disclosure. In addition, it would be appreciated that the objectives and advantages of the present disclosure may be implemented by means provided in the claims and a combination thereof.

### Solution to Problem

According to the present disclosure, an Alzheimer's disease diagnosis apparatus may include a processor, and a memory operably connected to the processor and storing at least one piece of code to be executed by the processor, wherein the memory stores code that, when executed by the processor, causes the processor to collect speech data of a speaker, extract features of the speech data, and generate, based on the features of the speech data, at least one of an Alzheimer's disease classification result and a cognitive function assessment score prediction result.

According to the present disclosure, the method of diagnosing Alzheimer's disease is performed by a processor of an Alzheimer's disease diagnosis apparatus, and may include collecting speech data of a speaker, extracting features of the speech data, and generating, based on the features of the speech data, at least one of an Alzheimer's disease classification result and a cognitive function assessment score prediction result.

In addition, other methods and systems for implementing the present disclosure, and a computer-readable recording medium having recorded thereon a computer program for executing the methods may be further provided.

Other aspects, features, advantages other than those described above will become apparent from the following drawings, claims, and detailed description of the present disclosure. The invention is defined by the appended claims.

### Advantageous Effects of Invention

According to the present disclosure, by analyzing speech characteristics of a speaker collected through a speech task and, based thereon, improving the diagnostic accuracy for Alzheimer's disease, it may be useful for early diagnosis and management of Alzheimer's disease.

Furthermore, by analyzing speech characteristics of a speaker collected through a speech task and, based thereon, accurately predicting a cognitive function assessment score, it may be useful for early diagnosis and management of Alzheimer's disease.

Effects of the present disclosure are not limited to the foregoing, and other unmentioned effects would be clearly understood by those skilled in the art from the following description.

### Brief Description of Drawings

FIG. 1 is a diagram illustrating an Alzheimer's disease diagnosis environment according to an embodiment.
FIG. 2 is a block diagram schematically illustrating a configuration of an Alzheimer's disease diagnosis apparatus according to an embodiment.
FIG. 3 is a block diagram schematically illustrating a configuration of a diagnosis management unit of the Alzheimer's disease diagnosis apparatus of FIG. 2.
FIG. 4 is a structural diagram schematically illustrating a structure of the diagnosis management unit illustrated in FIG. 3.
FIG. 5 is a diagram illustrating an example of a speech task for collecting speech data of a speaker, according to an embodiment.
FIG. 6 is a table showing a comparison of features of speech data between an Alzheimer's disease patient group and a normal group, according to an embodiment.
FIG. 7 is a table showing a comparison of features of speech data between Alzheimer's disease patients and healthy older adults for each speech task, according to an embodiment.
FIG. 8 is a table showing a comparison of performance between an Alzheimer's disease classification result and a cognitive function assessment score prediction result, according to an embodiment.
FIG. 9 is a block diagram schematically illustrating a configuration of an Alzheimer's disease diagnosis apparatus according to another embodiment.
FIGS. 10 to 12 are flowcharts for describing a method of diagnosing Alzheimer's disease according to an embodiment.

### Mode for the Invention

Advantages and features of the present disclosure and a method for achieving them will be apparent with reference to embodiments of the present disclosure described below together with the accompanying drawings.

Terms used herein are for describing particular embodiments and are not intended to limit the scope of the present disclosure. The singular expression also includes the plural meaning as long as it is not inconsistent with the context. In the present specification, it is to be understood that the terms such as "including," "having," and "comprising" are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added. Terms such as "first" or "second" may be used to describe various elements, but the elements should not be limited by the terms. These terms are used only to distinguish one element from another.

In addition, as used herein, terms such as "...er", "...or", or "... unit" denote a unit that performs at least one function or operation, which may be implemented as hardware or software or a combination thereof.

Hereinafter, embodiments of the present disclosure are described in detail with reference to the accompanying drawings, and the same or corresponding components are denoted by the same reference numerals when described with reference to the accompanying drawings, and thus, redundant descriptions thereof are omitted.

In the following embodiments, terms such as "first," "second," etc., are used only to distinguish one component from another, and such components must not be limited by these terms.

In the following embodiments, the singular expression also includes the plural meaning as long as it is not inconsistent with the context.

In the following embodiments, the terms "comprise," "include," "have," and the like specify the presence of stated features or components, but do not preclude the presence or addition of one or more other features or components.

When a certain embodiment may be differently implemented, particular operations may be performed differently from the sequence described herein. For example, two processes, which are successively described herein, may be substantially simultaneously performed, or may be performed in a process sequence opposite to a described process sequence.

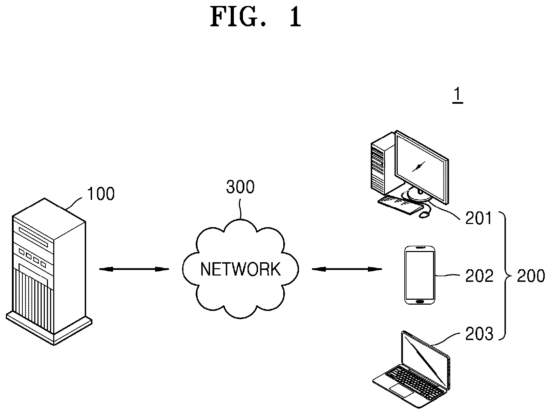

FIG. 1 is a diagram illustrating an Alzheimer's disease diagnosis environment according to an embodiment. Referring to FIG. 1, an Alzheimer's disease diagnosis environment 1 may include an Alzheimer's disease diagnosis apparatus 100, a user terminal 200, and a network 300.

The Alzheimer's disease diagnosis apparatus 100 may collect speech data of a speaker, from a speaker terminal (hereinafter, referred to as the user terminal 200) used by the speaker. The Alzheimer's disease diagnosis apparatus 100 may collect first to third speech data uttered by a speaker in response to first to third speech tasks. In an embodiment, the first speech task may include an instruction requesting the speaker's response to a preset question. Here, the first speech data may include speech data collected via an interview task. In an embodiment, the second speech task may involve outputting an audio narration of a preset story, and may include an instruction requesting the speaker to repeat the content of the audio narration. Here, the second speech data may include speech data collected via a repetition task. The third speech task may include an instruction requesting a recall of a preset story. Here, the third speech data may include speech data collected via a recall task.

The Alzheimer's disease diagnosis apparatus 100 may extract features from the collected speech data, that is, the first to third speech data. The Alzheimer's disease diagnosis apparatus 100 may separate the collected speech data into a speech section and a pause section, and extract features from the speech data included in the speech section. In an embodiment, the Alzheimer's disease diagnosis apparatus 100 may extract, from the speech data included in the speech section, at least one of frequency-related features, loudness-related features, temporal features, and spectrum features.

Based on the extracted features of the speech data, the Alzheimer's disease diagnosis apparatus 100 may generate at least one of an Alzheimer's disease classification result and a cognitive function assessment score prediction result. In an embodiment, before generating at least one of the Alzheimer's disease classification result and the cognitive function assessment score prediction result, the Alzheimer's disease diagnosis apparatus 100 may select features of the speech data. The Alzheimer's disease diagnosis apparatus 100 may use an analysis-of-variance (ANOVA) algorithm to select features of the speech data.

The Alzheimer's disease diagnosis apparatus 100 may generate at least one of the Alzheimer's disease classification result and the cognitive function assessment score prediction result by using a result of selecting features of the speech data. Here, in an embodiment, the cognitive function assessment score may include a mini-mental state examination (MMSE) score. The MMSE is one of the standardized test tools for assessing cognitive function, and may be used to diagnose neurological and mental disorders such as Alzheimer's disease. The MMSE is typically scored on a scale of 0 to 30, and a higher score may indicate better cognitive function. A score in the range of 27 to 30 is considered within the normal range, and may indicate that there are no cognitive impairments or problems. A score in the range of 21 to 26 represents mild cognitive impairment, where there may be minor cognitive problems. A score in the range of 11 to 20 represents moderate cognitive impairment, and may indicate the presence of significant cognitive impairments. A score in the range of 0 to 10 represents severe cognitive impairment, and severe cognitive impairments or dementia may be suspected.

The Alzheimer's disease diagnosis apparatus 100 may use an artificial intelligence algorithm to generate at least one of the Alzheimer's disease classification result and the cognitive function assessment score prediction result. Here, artificial intelligence (AI) is a field of computer engineering and information technology for researching a method for allowing computers to do thinking, learning, self-development or the like that can be done by human intelligence, and may refer to a process of causing a computer to imitate human intelligent behavior.

In addition, Al does not exist on its own, but is rather directly or indirectly connected with other fields of computer science. Particularly in modern times, attempts to introduce elements of Al into various fields of information technology and utilize them for problem-solving in those fields have been actively made.

Machine learning is an application of Al that gives computers the ability to automatically learn and improve from experience without explicit programs. In detail, machine learning is a technique for researching and building a system that performs learning based on empirical data, performs predictions, and improves its own performance, and algorithms therefor. The algorithms in machine learning may take a way of building specific models to derive predictions or decisions based on input data, rather than performing strictly defined static program instructions.

Both unsupervised learning and supervised learning may be used as machine learning methods for such AI networks. In addition, deep learning technology, which is a subfield of machine learning, may enable multi-step, deep-level learning based on data. Deep learning may refer to a set of machine learning algorithms for extracting key data from a plurality of pieces of data as the number of steps increases.

In an embodiment, the Alzheimer's disease diagnosis apparatus 100 may be implemented as an independent server, or an Alzheimer's disease diagnosis function provided by the Alzheimer's disease diagnosis apparatus 100 may be implemented as an application to be installed on the user terminal 200. In addition, the Alzheimer's disease diagnosis apparatus 100 may be a database server that provides data necessary for applying various Al algorithms.

The user terminal 200 may receive an Alzheimer's disease diagnosis service by accessing an Alzheimer's disease diagnosis application and/or an Alzheimer's disease diagnosis site provided by the Alzheimer's disease diagnosis apparatus 100.

The user terminal 200 may include a communication terminal capable of performing a function of a computing device (not shown), and may be, in addition to a desktop computer 201, a smart phone 202, and a notebook computer 203 that are operated by a user, a tablet personal computer (PC), a smart television (TV), a mobile phone, a personal digital assistant (PDA), a media player, a microserver, a global positioning system (GPS) device, an electronic book terminal, a digital broadcasting terminal, a navigation device, a kiosk, an MP3 player, a digital camera, a home appliance, and other mobile or non-mobile computing device, but is not limited thereto. In addition, the user terminal 200 may be a wearable terminal, such as a watch, glasses, a hair band, or a ring, which has a communication function and a data processing function. The user terminal 200 is not limited to the above description, and may be any terminal capable of web browsing.

The network 300 may serve to connect the Alzheimer's disease diagnosis apparatus 100 to the user terminal 200. The network 300 may include, for example, a wired network such as a local area network (LAN), a wide area network (WAN), a metropolitan area network (MAN), or an integrated services digital network (ISDN), or a wireless network such as a wireless LAN (WLAN), code-division multiple access (CDMA), or satellite communication, but the present disclosure is not limited thereto. In addition, the network 300 may transmit and receive information by using short-range communication and/or long-range communication. Here, the short-range communication may include Bluetooth, radio-frequency identification (RFID), Infrared Data Association (IrDA), ultra-wideband (UWB), ZigBee, and wireless fidelity (Wi-Fi), and the long-range communication may include code-division multiple access (CDMA), frequency-division multiple access (FDMA), time-division multiple access (TDMA), orthogonal FDMA (OFDMA), and single-carrier FDMA (SC-FDMA).

The network 300 may include connection of network elements, such as hubs, bridges, routers, or switches. The network 300 may include one or more connected networks, for example, a multi-network environment, including a public network, such as the Internet, and a private network, such as a secure corporate private network. Access to the network 300 may be provided through one or more wired or wireless access networks.

Furthermore, the network 300 may support controller area network (CAN) communication, vehicle-to-infrastructure (V2I) communication, vehicle-to-everything (V2X) communication, wireless access in vehicular environment (WAVE) communication, and an Internet-of-Things (IoT) network and/or 5G communication that allows distributed components, such as objects, to exchange and process information.

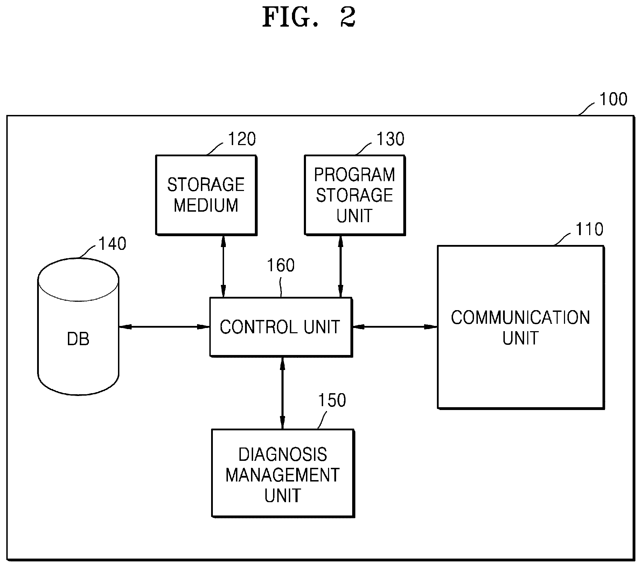

FIG. 2 is a block diagram schematically illustrating a configuration of an Alzheimer's disease diagnosis apparatus according to an embodiment. In the following description, redundant descriptions provided above with reference to FIG. 1 will be omitted. Referring to FIG. 2, the Alzheimer's disease diagnosis apparatus 100 may include a communication unit 110, a storage medium 120, a program storage unit 130, a database 140, a diagnosis management unit 150, and a control unit 160.

In conjunction with the network 300, the communication unit 110 may provide a communication interface necessary to provide signals, which are transmitted and received between the Alzheimer's disease diagnosis apparatus 100 and the user terminal 200, in the form of packet data. Furthermore, the communication unit 110 may serve to receive a certain information request signal from the user terminal 200, and transmit information processed by the diagnosis management unit 150 to the user terminal 200. Here, the communication interface refers to a medium that connects the Alzheimer's disease diagnosis apparatus 100 to the user terminal 200, and may include a path providing access such that the user terminal 200 may transmit and receive information after connecting to the Alzheimer's disease diagnosis apparatus 100. In addition, the communication unit 110 may be a device including hardware and software necessary for transmitting and receiving signals, such as control signals or data signals, through wired/wireless connection with other network devices.

The storage medium 120 performs a function of temporarily or permanently storing data processed by the control unit 160. Here, the storage medium 120 may include a magnetic storage medium or a flash storage medium, but the scope of the present disclosure is not limited thereto. The storage medium 120 may include an internal memory and/or an external memory, and may include a volatile memory, such as dynamic random-access memory (DRAM), static random-access memory (SRAM), or synchronous DRAM (SDRAM), nonvolatile memory such as a one-time programmable read-only memory (OTPROM), programmable read-only memory (PROM), erasable programmable read-only memory (EPROM), electrically erasable programmable read-only memory (EEPROM), mask read-only memory (ROM), flash ROM, NAND flash memory, or NOR flash memory, a flash drive such as a solid-state drive (SSD), a compact flash (CF) card, a Secure Digital (SD) card, a Micro-SD card, a Mini-SD card, an eXtreme Digital (XD) card, or a memory stick, or a storage device, such as a hard disk drive (HDD).

The program storage unit 130 stores control software that performs collecting speech data of a speaker, separating the collected speech data into a speech section and a pause section, extracting features of the speech data included in the speech section, selecting features of specific speech data from among the extracted features of the speech data, and generating at least one of an Alzheimer's disease classification result and a cognitive function assessment score prediction result by using the selected features.

The database 140 may include a management database that stores various pieces of information for diagnosing Alzheimer's disease. Speech tasks for collecting speech data may be stored in the management database. An algorithm for separating a speech section from collected speech data (e.g., voice studio 2.0) may be stored in the management database. An algorithm for extracting features of speech data (e.g., eGeMAPS) may be stored in the management database. An algorithm for selecting features of speech data (e.g., ANOVA) may be stored in the management database. An Al algorithm for generating at least one of an Alzheimer's disease classification result and a cognitive function assessment score prediction result may be stored in the management database.

In addition, the database 140 may include a user database that stores information about a user who will receive the Alzheimer's disease diagnosis service (i.e., the speaker described above). Here, the information about the user may include basic information about the user, such as the user's name, affiliation, personal information, gender, age, contact information, email, address, or photo, information about user authentication (login), such as an identifier (ID) (or an e-mail) or a password, and access-related information, such as a country of access, a location of access, information about a device used for access, or a network environment of access.

First to third speech data collected from a user for diagnosing Alzheimer's disease may be stored in the user database. In addition, the user database may store a user's unique information, information and/or a category history provided to a user who accessed the Alzheimer's disease diagnosis application or an Alzheimer's disease diagnosis site, information about environment settings by the user, information about resources used by the user, billing and payment information with respect to the user's resource usage.

The diagnosis management unit 150 may collect speech data of a speaker. The diagnosis management unit 150 may separate the collected speech data into a speech section and a pause section. The diagnosis management unit 150 may extract features of the speech data included in the speech section. The diagnosis management unit 150 may select features of specific speech data from among the extracted features of the speech data. The diagnosis management unit 150 may generate at least one of an Alzheimer's disease classification result and a cognitive function assessment score prediction result by using the selected features of the speech data.

The control unit 160 is a type of central processing unit and may control the overall operation of the Alzheimer's disease diagnosis apparatus 100 by executing control software stored in the program storage unit 130. The control unit 160 may include any type of device capable of processing data, such as a processor. Here, the 'processor' may refer to a hardware-embedded data processing device having a physically structured circuitry to perform functions represented by code or instructions included in a program. Examples of the hardware-embedded data processing device may include a processing device, such as a microprocessor, a central processing unit (CPU), a processor core, a multiprocessor, an application-specific integrated circuit (ASIC), and a field-programmable gate array (FPGA), but the present disclosure is not limited thereto.

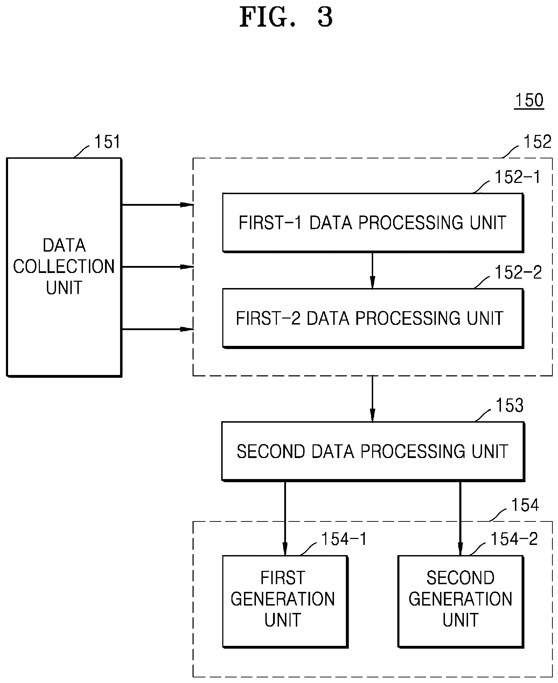

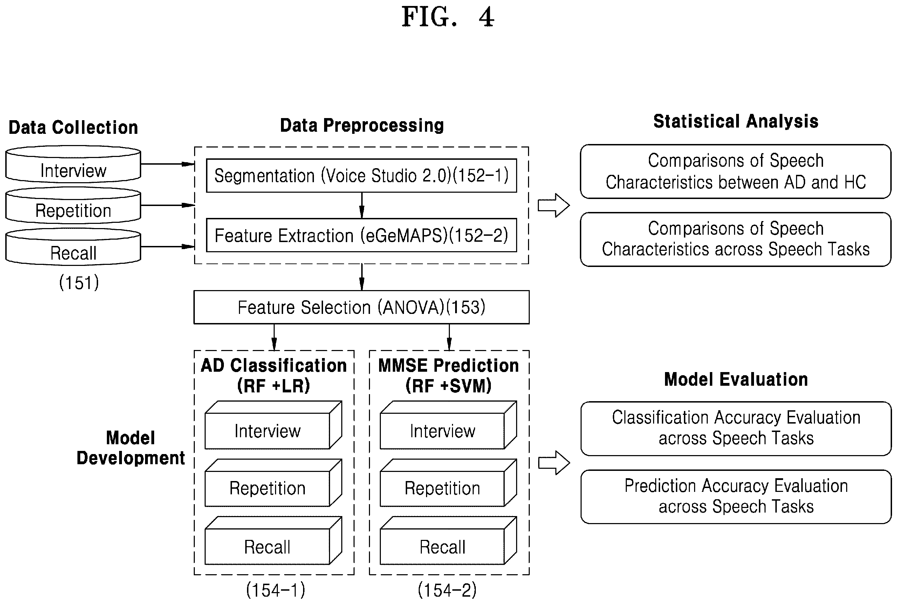

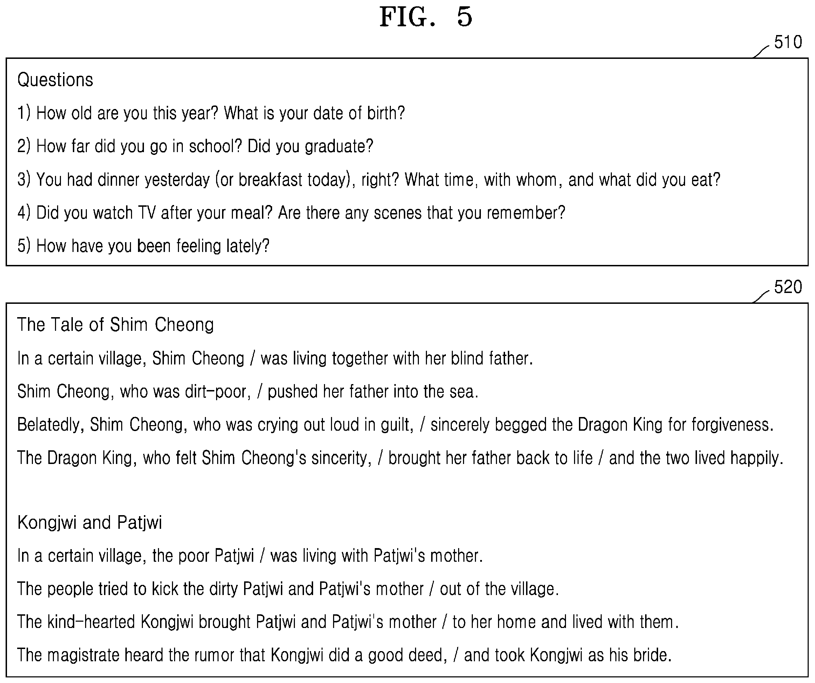

FIG. 3 is a block diagram schematically illustrating a configuration of the diagnosis management unit of the Alzheimer's disease diagnosis apparatus of FIG. 2, FIG. 4 is a structural diagram schematically illustrating a structure of the diagnosis management unit illustrated in FIG. 3, and FIG. 5 is a diagram illustrating an example of a speech task for collecting speech data of a speaker, according to an embodiment. In the following description, redundant descriptions provided above with reference to FIGS. 1 and 2 will be omitted. Referring to FIGS. 3 to 7, the diagnosis management unit 150 may include a data collection unit 151, a first data processing unit 152, a second data processing unit 153, and a generation unit 154.

The data collection unit 151 may collect speech data of a speaker. The data collection unit 151 may collect first to third speech data uttered by a speaker in response to first to third speech tasks. The first to third speech data collected by the data collection unit 151 may be stored in the database 140 as first to third speech files.

The data collection unit 151 may collect the first speech data uttered by the speaker in response to the first speech task that requests the speaker's response to one or more preset questions. 510 of FIG. 5 illustrates examples of questions included in the first speech task, for example, a question about age and date of birth, a question about educational background, a question about meal, a question about behavior after a meal, and a question about mood. In an embodiment, content related to the questions is not limited to the examples described above and may be changed.

The data collection unit 151 may collect the second speech data uttered by the speaker in response to the second speech task that outputs an audio narration of a preset story and requests the speaker to repeat the audio narration. 520 of FIG. 5 illustrates "The Tale of Shim Cheong" and "Kongjwi and Patjwi", as examples of preset stories included in the second speech task and the third speech task. In an embodiment, content related to the preset stories is not limited to the examples described above and may be changed. In an embodiment, the second speech data uttered by the speaker in response to the second speech task that outputs an audio narration of a preset story, which is read with pauses according to the delimiter ("/"), and requests the speaker to repeat the audio narration, may be collected.

The data collection unit 151 may collect the third speech data uttered by the speaker in response to the third speech task that requests a recall of the above-described story. In an embodiment, immediately after collecting the second speech data, that is, without a time delay, the data collection unit 151 may collect the third speech data of the speaker in response to the third speech task.

The first data processing unit 152 may extract features of the speech data, from the first to third speech data of the speaker. In an embodiment, the first data processing unit 152 may include a first-1 data processing unit 152-1 and a first-2 data processing unit 152-2.

The first-1 data processing unit 152-1 may separate the first to third speech data of the speaker into a speech section and a pause section.

To separate the speech data into the speech section and the pause section, the first-1 data processing unit 152-1 may use an algorithm such as voice studio 2.0. Voice studio 2.0 may be a program that separates a speech section via clicking and dragging. After a speech file is uploaded to the program, a speech section to be separated may be designated by dragging. When separation of a single speech file into a speech section and a pause section is completed, a timestamp corresponding to the speech section may be stored in a JSON file format. Based on timestamps in the corresponding file, a speech file corresponding to each speech section may be separated and stored by utilizing a Python library.

The first-2 data processing unit 152-2 may extract features of the first to third speech data included in the speech section. Here, the features of the speech data may include at least one of frequency-related features, loudness-related features, temporal features, and spectrum features.

The frequency-related features relate to a pitch of a voice, and may include a fundamental frequency (FO), jitter, and the like. The loudness-related features relate to an amplitude of a voice, and may include loudness, shimmer, and the like. The temporal features relate to a speech rate, and may include a duration of a voice, and the like. The spectrum features indicate how much energy exists for each of various frequencies in a voice having various amplitudes and frequencies, and may include mel-frequency cepstral coefficients (MFCCs), an alpha ratio, a Hammarberg index, and the like.

MFCC may be used for speech recognition and speech processing. MFCC mainly uses a mel-scale frequency band and may divide a frequency band with a mel filter bank. In this way, human auditory characteristics for a voice may be modeled. Alpha ratio may be used to evaluate an energy distribution in a frequency band of a voice. Alpha ratio may calculate an energy ratio by dividing a frequency band into a plurality of intervals. Hammarberg index may be used to evaluate voice quality and pronunciation characteristics. Compared to MFCC and alpha ratio, Hammarberg index may use a more general frequency band and calculate an energy distribution for a specific frequency band.

In an embodiment, an algorithm such as the extended Geneva Minimalistic Acoustic Parameter Set (eGeMAPS) may be used to extract features of speech data. A function for calculating and extracting frequency, loudness, speech duration, and spectrum features is embedded in the Python library opensmile, and features may be extracted by inputting, to the library, the first to third speech data included in the speech section. In an embodiment, when the eGeMAPS algorithm is applied, 88 speech features may be extracted per speech section.

Here, the 88 speech features may include fundamental frequency-related derived variables, loudness-related derived variables, spectral characteristic-related variables, voice quality-related variables, formant-related variables, and speech rate-related variables, as follows.

The fundamental frequency (FO)-related derived variables may include F0semitoneFrom27.5Hz_sma3nz_amean (mean value of F0 on a semitone frequency scale based on 27.5 Hz, representing voice pitch), FOsemitoneFrom27.5Hz_sma3nz_stddevNorm (normalized standard deviation of FO, representing the degree of variability in voice pitch), FOsemitoneFrom27.5Hz_sma3nz_percentile20.0 (20th percentile of FO), FOsemitoneFrom27.5Hz_sma3nz_percentile50.0 (50th percentile of FO), FOsemitoneFrom27.5Hz_sma3nz_percentile80.0 (80th percentile of FO), FOsemitoneFrom27.5Hz_sma3nz_pctlrange0-2 (range between 0 to 2 % of the entire FO), FOsemitoneFrom27.5Hz_sma3nz_meanRisingSlope (mean value of the FO rising slope), FOsemitoneFrom27.5Hz_sma3nz_stddevRisingSlope (standard deviation of the FO rising slope), FOsemitoneFrom27.5Hz_sma3nz_meanFallingSlope (mean value of the FO falling slope), FOsemitoneFrom27.5Hz_sma3nz_stddevFallingSlope (standard deviation of the FO falling slope), logReIF0-H1-H2_sma3nz_amean (mean energy ratio of the first FO harmonic (H 1) to the highest harmonic energy in the second harmonic frequency (H2)), logReIF0-H1-H2_sma3nz_stddevNorm (standard deviation of the energy ratio of the first FO harmonic (H 1) to the highest harmonic energy in the second harmonic frequency (H2)), logRelF0-H1-A3_sma3nz_amean (mean energy ratio of the first FO harmonic (H1) to the highest harmonic energy in the third formant range (A3)), and logReIF0-H1-A3_sma3nz_stddevNorm (standard deviation of the energy ratio of the first FO harmonic (H1) to the highest harmonic energy in the third formant range (A3)).

The loudness-related derived variables may include loudness_sma3_amean (mean loudness of the speech signal, representing voice volume), loudness_sma3_stddevNorm (standard deviation of the speech signal loudness, representing the degree of variability in voice volume), loudness_sma3_percentile20.0 (20th percentile of the speech signal loudness), loudness_sma3_percentile50.0 (50th percentile of the speech signal loudness), loudness_sma3_percentile80.0 (80th percentile of the speech signal loudness), loudness_sma3_pctlrange0-2 (range between 0 to 2 % of the entire speech signal loudness), loudness_sma3_meanRisingSlope (mean value of the speech signal loudness rising slope), loudness_sma3_stddevRisingSlope (standard deviation of the speech signal loudness rising slope), loudness_sma3_meanFallingSlope (mean value of the speech signal loudness falling slope), loudness_sma3_stddevFallingSlope (standard deviation of the speech signal loudness falling slope), and loudnessPeaksPerSec (number of loudness peaks of the speech signal measured per second).

The spatial characteristic-related variables may include spectralFlux_sma3_amean (mean value of the spectral flux (an indicator representing the difference in the frequency spectrum between consecutive speech frames, which is used to measure voice changes and energy fluctuations), spectralFlux_sma3_stddevNorm (standard deviation of the spectral flux), mfcc1_sma3_amean (mean value of the first coefficient of MFCCs, which is one of the main speech features representing frequency details of a speech signal), mfcc1_sma3_stddevNorm (standard deviation of the first coefficient of the MFCC), mfcc2_sma3_amean (mean value of the second coefficient of the MFCC), mfcc2_sma3_stddevNorm (standard deviation of the second coefficient of the MFCC), mfcc3_sma3_amean (mean value of the third coefficient of the MFCC), mfcc3_sma3_stddevNorm (standard deviation of the third coefficient of the MFCC), mfcc4_sma3_amean (mean value of the fourth coefficient of the MFCC), mfcc4_sma3_stddevNorm (standard deviation of the fourth coefficient of the MFCC), alphaRatioV_sma3nz_amean (mean value of the alpha ratio, which signifies the energy distribution within the harmonic frequency band of the speech signal), alphaRatioV_sma3nz_stddevNorm (standard deviation of the alpha ratio), hammarbergIndexV_sma3nz_amean (mean value of the Hammarberg index, which is an indicator for evaluating the flatness and energy distribution of a voice by measuring the spectral characteristics of the speech signal), hammarbergIndexV_sma3nz_stddevNorm (standard deviation of the Hammarberg index), slopeVO-500_sma3nz_amean (mean of the slope values extracted from the frequency range of 0 Hz to 500 Hz), slopeVO-500_sma3nz_stddevNorm (standard deviation of the slope values extracted from the frequency range of 0 Hz to 500 Hz), slopeV500-1500_sma3nz_amean (mean of the slope values extracted from the frequency range of 500 Hz to 1500 Hz), slopeV500-1500_sma3nz_stddevNorm (standard deviation of the slope values extracted from the frequency range of 500 Hz to 1500 Hz), spectralFluxV_sma3nz_amean (mean value of the spectral flux of a voiced segment within given audio data), spectralFluxV_sma3nz_stddevNorm (standard deviation of a voiced segment within given audio data), mfcc1V_sma3nz_amean (mean of the first coefficient of the MFCC of a voiced segment within given audio data), mfcc1V_sma3nz_stddevNorm (standard deviation of the first coefficient of the MFCC of a voiced segment within given audio data), mfcc2V_sma3nz_amean (mean of the second coefficient of the MFCC of a voiced segment within given audio data), mfcc2V_sma3nz_stddevNorm (standard deviation of the second coefficient of the MFCC of a voiced segment within given audio data), mfcc3V_sma3nz_amean (mean of the third coefficient of the MFCC of a voiced segment within given audio data), mfcc3V_sma3nz_stddevNorm (standard deviation of the third coefficient of the MFCC of a voiced segment within given audio data), mfcc4V_sma3nz_amean (mean of the fourth coefficient of the MFCC of a voiced segment within given audio data), mfcc4V_sma3nz_stddevNorm (standard deviation of the fourth coefficient of the MFCC of a voiced segment within given audio data), alphaRatioUV_sma3nz_amean (mean value of the alpha ratio of a voiced segment within given audio data), hammarberglndexUV_sma3nz_amean (mean value of the Hammarberg index of a voiced segment within given audio data), slopeUVO-500_sma3nz_amean (mean of the slope values extracted from the frequency range of 0 Hz to 500 Hz of a voiced segment within given audio data), and slopeUV500-1500_sma3nz_amean (mean of the slope values extracted from the frequency range of 500 Hz to 1500 Hz of a voiced segment within given audio data).

The voice quality-related variables may include jitterLocal_sma3nz_amean (mean value of jitter, which signifies the degree of irregularity in frequency for each vocalization cycle), jitterLocal_sma3nz_stddevNorm (standard deviation of jitter), shimmerLocaldB_sma3nz_amean (mean value of shimmer, which signifies the degree of irregularity in voice intensity for each vocalization cycle), shimmerLocaldB_sma3nz_stddevNorm (standard deviation of shimmer), HNRdBACF_sma3nz_amean (mean value of the harmonic-to-noise Ratio (HNR), which provides information about the overall noise characteristics of a voice), and HNRdBACF_sma3nz_stddevNorm (standard deviation of the HNR).

The formant-related variables may include F1frequency_sma3nz_amean (mean value of the first formant frequency), F1frequency_sma3nz_stddevNorm (standard deviation of the first formant frequency), F1bandwidth_sma3nz_amean (mean value of the first formant bandwidth), F1bandwidth_sma3nz_stddevNorm (standard deviation of the first formant bandwidth), F1amplitudeLogRelFO_sma3nz_amean (mean of the value representing the first formant amplitude converted to a logarithm and expressed as a value relative to FO), F1amplitudeLogReIF0_sma3nz_stddevNorm (standard deviation of the value representing the first formant amplitude converted to a logarithm and expressed as a value relative to FO), F2frequency_sma3nz_amean (mean value of the second formant frequency), F2frequency_sma3nz_stddevNorm (standard deviation of the second formant frequency), F2bandwidth_sma3nz_amean (mean value of the second formant bandwidth), F2bandwidth_sma3nz_stddevNorm (standard deviation of the second formant bandwidth), F2amplitudeLogRelFO_sma3nz_amean (mean of the value representing the second formant amplitude converted to a logarithm and expressed as a value relative to FO), F2amplitudeLogReIF0_sma3nz_stddevNorm (standard deviation of the value representing the second formant amplitude converted to a logarithm and expressed as a value relative to FO), F3frequency_sma3nz_amean (mean value of the third formant frequency), F3frequency_sma3nz_stddevNorm (standard deviation of the third formant frequency), F3bandwidth_sma3nz_amean (mean value of the third formant bandwidth), F3bandwidth_sma3nz_stddevNorm (standard deviation of the third formant bandwidth), F3amplitudeLogRelFO_sma3nz_amean (mean of the value representing the third formant amplitude converted to a logarithm and expressed as a value relative to FO), and F3amplitudeLogReIF0_sma3nz_stddevNorm (standard deviation of the value representing the third formant amplitude converted to a logarithm and expressed as a value relative to FO).

The speech rate-related variables may include VoicedSegmentsPerSec (the number of voiced segments of the audio data measured per second), MeanVoicedSegmentLengthSec (mean length of voiced segments of the audio data measured per second), StddevVoicedSegmentLengthSec (standard deviation of the length of voiced segments of the audio data measured per second), MeanUnvoicedSegmentLength (mean length of unvoiced segments of the audio data measured per second), and StddevUnvoicedSegmentLength (standard deviation of the length of unvoiced segments of the audio data measured per second).

The second data processing unit 153 may select features of the speech data that satisfy a preset condition, from a feature extraction result of the speech data. In other words, the second data processing unit 153 may select features of the speech data to be used for generating an Alzheimer's disease classification result and a cognitive function assessment score prediction result.

To select the features of the speech data, the second data processing unit 153 may use an ANOVA algorithm. In statistics, ANOVA may include a hypothesis-testing method used to compare the means of two or more groups by using an F-distribution, which is derived from a comparison between a within-group variance and a between-group variance, wherein the between-group variance is resulting from the differences between the means of the respective groups and the overall total mean.

The second data processing unit 153 may analyze 88 correlations between the features of the speech data and a presence or absence of Alzheimer's disease through repetitive measurements of variance analysis, by using the features of the speech data extracted by the first-2 data processing unit 152-2 as independent variables and the presence or absence of Alzheimer's disease as a dependent variable. The second data processing unit 153 may select features of the speech data that are below a preset significance level (ρ<0.001), from the result of analyzing the 88 correlations, and then use the selected features to generate an Alzheimer's disease classification result and a cognitive function assessment score prediction result.

The generation unit 154 may generate at least one of an Alzheimer's disease classification result and a cognitive function assessment score prediction result, based on the features of the speech data selected by the second data processing unit 153. In an embodiment, the generation unit 154 may simultaneously generate an Alzheimer's disease classification result and a cognitive function assessment score prediction result. The generation unit 154 may include a first generation unit 154-1 and a second generation unit 154-2.

The first generation unit 154-1 may generate an Alzheimer's disease classification result corresponding to the features of the speech data that are below the preset significance level, by using a first deep neural network model that is pre-trained to classify a presence or absence of Alzheimer's disease in response to features of speech data. Here, the first deep neural network model may be a model trained in a supervised learning manner with training data including features of speech data as an input and a presence or absence of Alzheimer's disease as a label.

The first generation unit 154-1 may train an initially set first deep neural network model in a supervised learning manner by using labeled training data. Here, the initially set first deep neural network model is an initial model designed to be configured as a model capable of classifying a presence or absence of Alzheimer's disease, in a state where its parameter values are set to arbitrary initial values. As the initial model is trained with the above-described training data, its parameter values are optimized, such that the initial model is completed as a classification model capable of accurately classifying a presence or absence of Alzheimer's disease.

In detail, the first generation unit 154-1 is a weighted voting classifier, and may classify a presence or absence of Alzheimer's disease based on features of speech data by combining random forest and logistic regression. A weight may be added according to the performance of the classifier. Hyperparameters may be adjusted on the training data through 10-fold cross-validation, and the resulting model may be evaluated on test data. To evaluate and compare the performance of different speech tasks, accuracy, precision, sensitivity, specificity, and an F1-score may be measured.

The second generation unit 154-2 may generate a cognitive function assessment score prediction result corresponding to features of speech data that are below the preset significance level, by using a second deep neural network model that is pre-trained to predict a cognitive function assessment score in response to features of speech data. Here, the second deep neural network model may be a model trained in a supervised learning manner with training data including features of speech data as an input and a cognitive function assessment score as a label.

The second generation unit 154-2 may train an initially set second deep neural network model in a supervised learning manner by using labeled training data. Here, the initially set second deep neural network model is an initial model designed to be configured as a model capable of predicting a cognitive function assessment score, in a state where its parameter values are set to arbitrary initial values. As the initial model is trained with the above-described training data, its parameter values are optimized, such that the initial model is completed as a classification model capable of accurately predicting a cognitive function assessment score.

In detail, the second generation unit 154-2 may be constructed with the same methodology as the first generation unit 154-1. The second generation unit 154-2 is a weighted regression model, and may be constructed by combining a random forest and a support vector machine.

In an embodiment, the generation unit 154 may generate three Alzheimer's disease classification results and three cognitive function assessment score prediction results. Based on a result of selecting features of the speech section of the first speech data, the generation unit 154 may generate a first Alzheimer's disease classification result and a first cognitive function assessment score prediction result. Based on a result of selecting features of the speech section of the second speech data, the generation unit 154 may generate a second Alzheimer's disease classification result and a second cognitive function assessment score prediction result. Based on a result of selecting features of the speech section of the third speech data, the generation unit 154 may generate a third Alzheimer's disease classification result and a third cognitive function assessment score prediction result.

In an alternative embodiment, the generation unit 154 may generate an Alzheimer's disease classification result based on a result of selecting features of the speech sections of the first speech data and the second speech data. Based on an Alzheimer's disease classification result, the generation unit 154 may determine whether to execute generation of a cognitive function assessment score prediction result. In an embodiment, when an Alzheimer's disease classification result for at least one of the features of the first speech data and the second speech data is generated as a first value ("1", Alzheimer's disease), the generation unit 154 may determine to execute generation of a cognitive function assessment score prediction result. However, when the Alzheimer's disease classification result for the features of the first speech data and the second speech data is generated as a second value ("0", normal), the generation unit 154 may determine not to execute generation of a cognitive function assessment score prediction result. When it is determined to execute generation of a cognitive function assessment score prediction result, the generation unit 154 may generate a cognitive function assessment score prediction result based on a result of selecting features of the speech section of the third speech data, excluding the features of the first speech data and the second speech data.

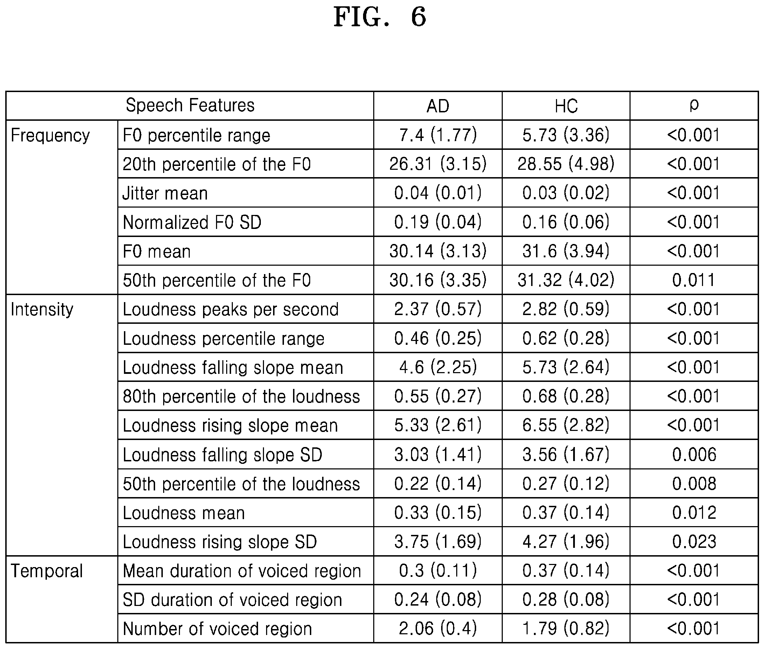

FIG. 6 is a table showing a comparison of features of speech data between an Alzheimer's disease patient group and a normal group, according to an embodiment. FIG. 6 may include an experimental result comparing features by using speech data collected from a plurality of Alzheimer's disease patients and a plurality of healthy older adults as a normal group. Referring to FIG. 6, AD (Alzheimer's disease) represents Alzheimer's disease patients, HC (healthy older adults) represents healthy older adults, and ρ represents a correlation coefficient in statistics.

First, in the frequency-related features, the mean FO (AD=30.14±3.13, HC=31.6±3.94, ρ<0.001), the 20th percentile of FO (AD=26.31±3.15, HC=28.55±4.98, ρ<0.001), and the 50th percentile of FO (AD=30.16±3.35, HC=31.32±4.02, ρ=0.011) indicate the pitch of a voice. In all cases, the values for the AD patients are lower than those for the healthy older adults. The FO percentile range indicates that the FO range of the AD patients is larger than that of the healthy older adults (AD=7.4±1.77, HC=5.73±3.36, ρ<0.001), and a high standard deviation of FO indicates that the FO variation of the AD patients is higher than that of the healthy older adults (AD=0.19±0.04, HC=0.16±0.06, ρ<0.001). The AD patients exhibit greater jitter than healthy older adults (AD=0.04±0.01, HC=0.03±0.02, ρ<0.001), which indicates high variability in pitch. In summary, the frequency-related features show that AD patients have a lower voice pitch and higher pitch variability compared to healthy older adults.

For Alzheimer's disease patients, the features related to voice loudness are as follows. The 80th percentile loudness (AD=0.55±0.27, HC=0.68±0.28, ρ<0.001), the 50th percentile loudness (AD=0.22±0.14, HC=0.27±0.12, ρ=0.008), and the mean loudness (AD=0.33±0.14, HC=0.37±0.14, ρ=0.012) were lower. The loudness peaks per second (AD=2.37±0.57, HC=2.82±0.59, ρ<0.001), the loudness percentile range (AD=0.46±0.25, HC=0.62±0.28, ρ<0.001), the loudness falling slope (AD=4.6±2.25, HC=5.73±2.64, ρ<0.001), and the loudness rising slope (AD=5.33±2.61, HC=6.55±2.82, ρ<0.001) all indicate loudness variability, and these were reduced in the AD patients. It may be seen that the loudness-related features show that AD patients have reduced voice loudness and speak monotonously when compared to healthy older adults.

In terms of temporal features, the AD patients had a shorter duration of voiced regions compared to healthy older adults (AD=0.3±0.11, HC=0.37±0.14, ρ<0.001). In addition, the number of voiced regions was greater compared to that of the healthy older adults (AD=2.06±0.4, HC=1.79±0.82, ρ<0.001). This suggests that AD patients have difficulty maintaining speech, speak at a lower rate than healthy older adults, and exhibit more temporary pauses and hesitations.

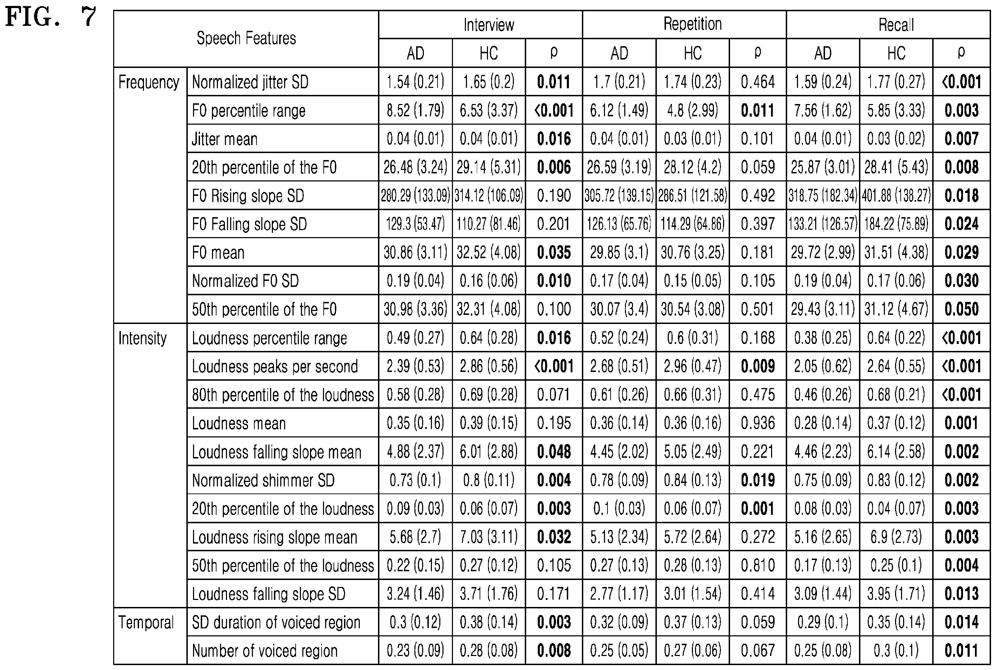

FIG. 7 is a table showing a comparison of features of speech data between Alzheimer's disease patients and healthy older adults for each speech task, according to an embodiment.

Referring to FIG. 7, with respect to frequency-related features, a specific trend was observed in voices of Alzheimer's disease patients, regardless of the speech task. In all speech tasks, the Alzheimer's disease patients exhibited a lower voice pitch (e.g., mean FO, and 20th and 50th percentiles) and greater pitch variability (e.g., FO percentile range and normalized FO standard deviation). However, in the interview task (first speech data) and the repetition task (second speech data), this trend was not statistically significant. However, in the recall task (third speech data), most of the features showed statistical significance. Furthermore, in the previous analysis conducted regardless of the speech task, there was no significant difference between the FO rising slope and the FO falling slope. However, after separating the data by speech task, it was found that these features were significantly different in the recall task.

With respect to the loudness-related features, a consistent trend was also observed regardless of the speech task. In all speech tasks, the Alzheimer's disease patients exhibited lower loudness (mean loudness, and 20th, 50th, and 80th percentiles) and spoke monotonously (loudness percentile range, loudness peaks per second, loudness rising slope, and loudness falling slope). However, in the interview task (first speech data) and the repetition task (second speech data), most of the loudness-related features did not show a statistically significant difference. On the other hand, in the recall task (third speech data), the difference was statistically significant.

With respect to the temporal features, the memory task did not show excellent discriminative power. The Alzheimer's disease patients had a shorter duration of voiced regions and more temporary pauses in the interview task (first speech data) and the recall task (third speech data).

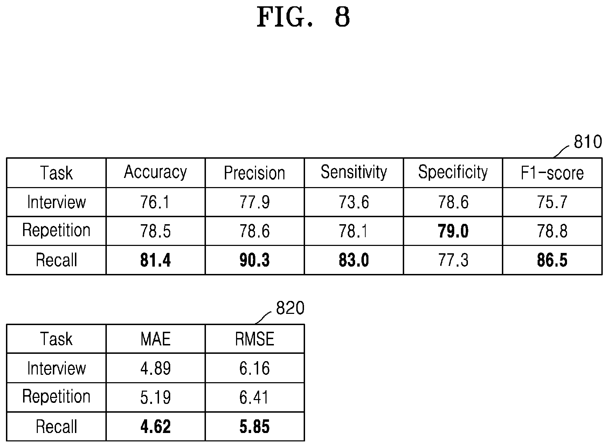

FIG. 8 is a table showing a comparison of performance between an Alzheimer's disease classification result and a cognitive function assessment score prediction result, according to an embodiment.

Referring to FIG. 8, 810 shows a comparison of the performance of a first deep neural network model (Alzheimer's disease classification model) across first speech data (interview task), second speech data (repetition task), and third speech data (recall task). The recall task (third speech data), with an accuracy of 81.4 %, showed superior performance to all other speech tasks, including the interview (first speech data) and the repetition task (second speech data). The repetition task (second speech data) achieved the second-highest accuracy of 78.5 %, and the interview task (first speech data) achieved an accuracy of 76.1 %. The first deep neural network model used a weighted evaluation classifier, which is a combination of logistic regression and random forest, and the best results are indicated in bold text.

In addition, 820 shows a comparison of the performance of a second deep neural network model (cognitive function assessment (e.g., MMSE) score prediction model) across the first speech data (interview task), the second speech data (repetition task), and the third speech data (recall task). The mean absolute error (MAE) and the root mean square error (RMSE) are prediction error measurement indicators, where the lower these indicators are, the higher the predictability is, and the best results are indicated in bold text.

As a result of investigating acoustic characteristics of voices of the AD patients and the healthy older adults from FIGS. 6 to 8, and investigating how these characteristics change under different speech tasks and memory loads, it was found that the AD patients had distinct voice characteristics from the healthy older adults, such as a low voice pitch, increased pitch variability, low loudness, monotonous loudness, and a low speech rate. In addition, it was shown that the voice characteristics of Alzheimer's disease may be emphasized when a high memory load is applied. In particular, it was indicated that the acoustic characteristics of AD voices may differ significantly depending on the speech task, and the recall task, which includes a high memory load, produced the most distinct difference. In addition, the recall task with a high memory load showed superior performance in the Alzheimer's classification model and the cognitive function assessment score prediction model than other tasks with a low memory load.

From this, it may be seen that the Alzheimer's disease diagnosis apparatus 100 according to an embodiment may be useful for early diagnosis and management of Alzheimer's disease by analyzing the speech characteristics of a speaker and, based thereon, accurately predicting the diagnostic accuracy for Alzheimer's disease and a cognitive function assessment score.

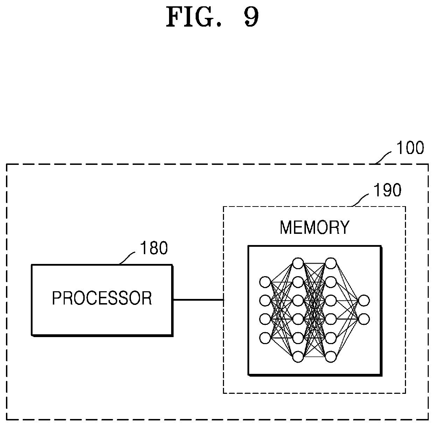

FIG. 9 is a block diagram schematically illustrating a configuration of an Alzheimer's disease diagnosis apparatus according to another embodiment. In the following description, redundant descriptions provided above with reference to FIGS. 1 to 8 will be omitted. Referring to FIG. 9, the Alzheimer's disease diagnosis apparatus 100 according to another embodiment may include a processor 170 and a memory 180.

In an embodiment, the processor 170 may process functions performed by the communication unit 110, the storage medium 120, the program storage unit 130, the database 140, the diagnosis management unit 150, and the control unit 160 that are illustrated in FIGS. 2 and 3.

The processor 170 may control the overall operation of the Alzheimer's disease diagnosis apparatus 100. Here, the 'processor' may refer to a hardware-embedded data processing device having a physically structured circuitry to perform functions represented by code or instructions included in a program. Examples of the hardware-embedded data processing device may include a processing device, such as a microprocessor, a CPU, a processor core, a multiprocessor, an ASIC, and an FPGA, but the scope of the present disclosure is not limited thereto.

The memory 180 may be operatively connected to the processor 170 and may store at least one piece of code associated with an operation performed by the processor 170.

In addition, the memory 180 may perform a function of temporarily or permanently storing data processed by the processor 170, and may include data constructed with the database 140. Here, the memory 180 may include a magnetic storage medium or a flash storage medium, but the scope of the present disclosure is not limited thereto. The memory 180 may include an internal memory and/or an external memory, and may include a volatile memory, such as DRAM, SRAM, or SDRAM, a nonvolatile memory such as OTPROM, PROM, EPROM, EEPROM, mask ROM, flash ROM, NAND flash memory, or NOR flash memory, a flash drive such as an SSD, a CF card, an SD card, a Micro-SD card, a Mini-SD card, an XD card, or a memory stick, or a storage device, such as an HDD.

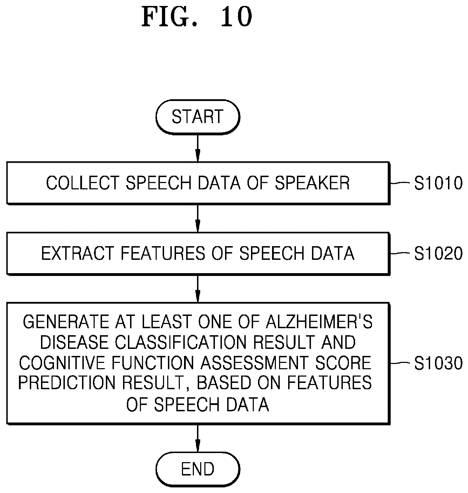

FIG. 10 is a flowchart for describing a method of diagnosing Alzheimer's disease according to an embodiment. In the following description, redundant descriptions provided above with reference to FIGS. 1 to 9 will be omitted. A method of diagnosing Alzheimer's disease according to an embodiment will be described assuming that it is performed by the processor 170 in the Alzheimer's disease diagnosis apparatus 100 with the help of peripheral components.

Referring to FIG. 10, in operation S1010, the processor 170 may collect speech data of a speaker. The processor 170 may collect first speech data uttered by the speaker in response to a first speech task that requests the speaker's response to one or more preset questions. Here, the first speech data may include speech data collected via an interview task. The processor 170 may collect second speech data uttered by the speaker in response to a second speech task that outputs an audio narration of a preset story and requests the speaker to repeat the audio narration. Here, the second speech data may include speech data collected via a repetition task. The processor 170 may collect third speech data uttered by the speaker in response to a third speech task that requests a recall of the above-described preset story. Here, the third speech data may include speech data collected via a recall task.

In operation S1020, the processor 170 may extract features of the collected speech data. Here, before extracting the features of the speech data, the processor 170 may separate the collected speech data into a speech section and a pause section. The processor 170 may extract at least one of frequency-related features, loudness-related features, temporal features, and spectrum features from the speech data included in the separated speech section. In an alternative embodiment, after extracting the features of the speech data, the processor 170 may select features of the speech data to be used for generating an Alzheimer's disease classification result and a cognitive function assessment score prediction result. To select the features of the speech data, the processor 170 may analyze correlations between the features of the speech data and a presence or absence of Alzheimer's disease through repetitive measurements of variance analysis, by using the features of the speech data as independent variables and the presence or absence of Alzheimer's disease as a dependent variable. The processor 170 may select features of the speech data that are below a preset significance level (e.g., ρ<0.001) from a result of analyzing the correlations.

In operation S1030, the processor 170 may generate at least one of an Alzheimer's disease classification result and a cognitive function assessment score prediction result, based on the selected features of the speech data. The processor 170 may simultaneously generate an Alzheimer's disease classification result and a cognitive function assessment score prediction result corresponding to the features of the speech data that are below the preset significance level. The processor 170 may generate an Alzheimer's disease classification result corresponding to the features of the speech data that are below the preset significance level, by using a first deep neural network model that is pre-trained to classify a presence or absence of Alzheimer's disease in response to features of speech data. Here, the first deep neural network model may be a model trained in a supervised learning manner with training data including features of speech data as an input and a presence or absence of Alzheimer's disease as a label. The processor 170 may generate a cognitive function assessment score prediction result corresponding to features of speech data that are below the preset significance level, by using a second deep neural network model that is pre-trained to predict a cognitive function assessment score in response to features of speech data. Here, the second deep neural network model may be a model trained in a supervised learning manner with training data including features of speech data as an input and a cognitive function assessment score as a label.

Furthermore, the processor 170 may provide the user terminal 200 with at least one of the Alzheimer's disease classification result and the cognitive function assessment score prediction result. When providing the user terminal 200 with at least one of the Alzheimer's disease classification result and the cognitive function assessment score prediction result, the processor 170 may provide instructions that a user may perform to prevent Alzheimer's disease (e.g., exercise or eating habits).

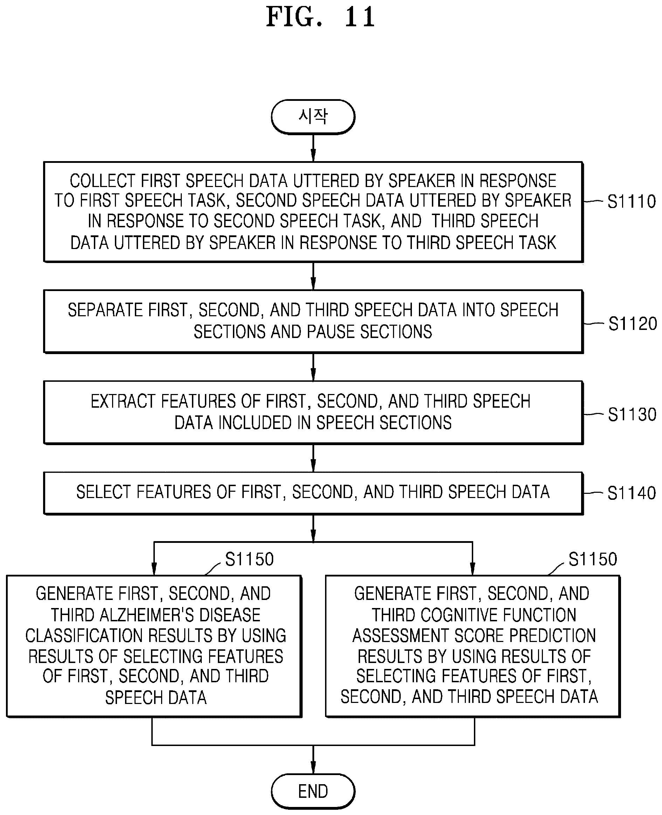

FIG. 11 is a flowchart for describing a method of diagnosing Alzheimer's disease according to another embodiment. In the following description, redundant descriptions provided above with reference to FIGS. 1 to 10 will be omitted. A method of diagnosing Alzheimer's disease according to an embodiment will be described assuming that it is performed by the processor 170 in the Alzheimer's disease diagnosis apparatus 100 with the help of peripheral components.

Referring to FIG. 11, in operation S1110, the processor 170 may collect first speech data uttered by a speaker in response to a first speech task, second speech data uttered by the speaker in response to a second speech task, and third speech data uttered by the speaker in response to a third speech task.

In operation S1120, the processor 170 may separate each of the first, second, and third speech data into a speech section and a pause section.

In operation S1130, the processor 170 may extract features of speech data, including at least one of frequency-related features, loudness-related features, temporal features, and spectrum features, from each of the first, second, and third speech data included in the speech sections. For example, the processor 170 may extract 88 features of speech data from each of the speech sections of the first, second, and third speech data.

In operation S1140, the processor 170 may select features of the first, second, and third speech data included in the speech sections. To select the features of the speech data, the processor 170 may analyze correlations between the features of the first, second, and third speech data and a presence or absence of Alzheimer's disease through repetitive measurements of variance analysis, by using the features of the first, second, and third speech data as independent variables and the presence or absence of Alzheimer's disease as a dependent variable. The processor 170 may select features of the first, second, and third speech data that are below a preset significance level (e.g., ρ<0.001) from a result of analyzing the correlations.

In operation S1150, the processor 170 may generate first, second, and third Alzheimer's disease classification results by using results of selecting features of the first, second, and third speech data, and generate first, second, and third cognitive function assessment score prediction results by using results of selecting features of the first, second, and third speech data.

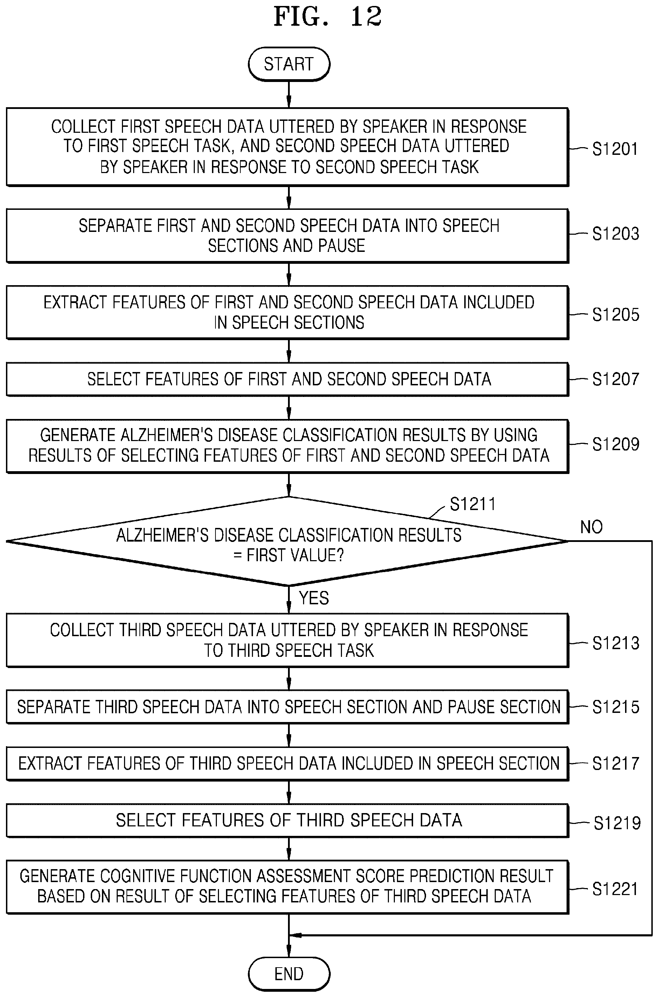

FIG. 12 is a flowchart for describing a method of diagnosing Alzheimer's disease according to yet another embodiment. In the following description, redundant descriptions provided above with reference to FIGS. 1 to 11 will be omitted. A method of diagnosing Alzheimer's disease according to an embodiment will be described assuming that it is performed by the processor 170 in the Alzheimer's disease diagnosis apparatus 100 with the help of peripheral components.

Referring to FIG. 12, in operation S1201, the processor 170 may collect first speech data uttered by a speaker in response to a first speech task, and second speech data uttered by the speaker in response to a second speech task.

In operation S1203, the processor 170 may separate the first and second speech data into speech sections and pause sections.

In operation S1205, the processor 170 may extract features of the first and second speech data included in the speech sections. The processor 170 may extract features of speech data, including at least one of frequency-related features, loudness-related features, temporal features, and spectrum features, from each of the first, second, and third speech data included in the speech sections. For example, the processor 170 may extract 88 features of speech data from each of the speech sections of the first and second speech data.

In operation S1207, the processor 170 may select features of the first and second speech data. To select the features of the speech data, the processor 170 may analyze correlations between the features of the first and second speech data and a presence or absence of Alzheimer's disease through repetitive measurements of variance analysis, by using the features of the first, second, and third speech data as independent variables and the presence or absence of Alzheimer's disease as a dependent variable. The processor 170 may select features of the first and second speech data that are below a preset significance level (e.g., ρ<0.001) from a result of analyzing the correlations.

In operation S1209, the processor 170 may generate Alzheimer's disease classification results by using results of selecting features of the first and second speech data. The processor 170 may simultaneously generate an Alzheimer's disease classification result and a cognitive function assessment score prediction result corresponding to the features of the speech data that are below the preset significance level. The processor 170 may generate an Alzheimer's disease classification result corresponding to the features of the speech data that are below the preset significance level, by using a first deep neural network model that is pre-trained to classify a presence or absence of Alzheimer's disease in response to features of speech data. Here, the first deep neural network model may be a model trained in a supervised learning manner with training data including features of speech data as an input and a presence or absence of Alzheimer's disease as a label.

In operation S1211, the processor 170 may determine whether the Alzheimer's disease classification results, which are generated by using the results of selecting features of the first and second speech data, are a first value. Here, the first value may indicate Alzheimer's disease. When the Alzheimer's disease classification results generated by using the results of selecting features of the first and second speech data are not the first value, that is, when they are a second value (normal), the processor 170 may terminate the process.

In operation S1213, when the Alzheimer's disease classification results generated by using the results of selecting features of the first and second speech data are the first value, the processor 170 may collect third speech data uttered by the speaker in response to a third speech task. In an embodiment, when an Alzheimer's disease classification result generated by using a result of selecting features of at least one of the first speech data and the second speech data is the first value, the processor 170 may collect third speech data uttered by the speaker in response to the third speech task.

In operation S1215, the processor 170 may separate the third speech data into a speech section and a pause section.

In operation S1217, the processor 170 may extract features of the third speech data included in the speech section. The processor 170 may extract features of speech data, including at least one of frequency-related features, loudness-related features, temporal features, and spectrum features, from the third speech data included in the speech section. For example, the processor 170 may extract 88 features of speech data from the speech section of the third speech data.

In operation S1219, the processor 170 may select features of the third speech data. To select the features of the speech data, the processor 170 may analyze correlations between the features of the third speech data and a presence or absence of Alzheimer's disease through repetitive measurements of variance analysis, by using the features of the third speech data as independent variables and the presence or absence of Alzheimer's disease as a dependent variable. The processor 170 may select features of the third speech data that are below a preset significance level (e.g., ρ<0.001) from a result of analyzing the correlations.

In operation S1221, the processor 170 may generate a cognitive function assessment score prediction result based on a result of selecting features of the third speech data. The processor 170 may generate a cognitive function assessment score prediction result corresponding to the features of the third speech data that are below the preset significance level, by using a second deep neural network model that is pre-trained to predict a cognitive function assessment score in response to features of speech data. Here, the second deep neural network model may be a model trained in a supervised learning manner with training data including features of speech data as an input and a cognitive function assessment score as a label.

Compared to FIGS. 10 and 11, the method of FIG. 12 may perform an earlier diagnosis of Alzheimer's disease more quickly. In FIGS. 10 and 11, the features of the first to third speech data are extracted and selected to generate the Alzheimer's disease classification results and the cognitive function assessment score prediction results. On the other hand, in FIG. 12, an earlier diagnosis of Alzheimer's disease may be performed more quickly by extracting and selecting features of the first and second speech data, and only when the speaker is classified as an Alzheimer's disease patient, extracting and selecting features of the third speech data to generate a cognitive function assessment score prediction result.

The embodiments of the present disclosure described above may be implemented as a computer program that may be executed through various components on a computer, and such a computer program may be recorded in a computer-readable medium. In this case, the medium may include a magnetic medium, such as a hard disk, a floppy disk, or a magnetic tape, an optical recording medium, such as a compact disc ROM (CD-ROM) or a digital video disc (DVD), a magneto-optical medium, such as a floptical disk, and a hardware device specially configured to store and execute program instructions, such as ROM, RAM, or flash memory.

In addition, the computer program may be specially designed and configured for the present disclosure or may be well-known to and usable by those skilled in the art of computer software. Examples of the computer program may include not only machine code, such as code made by a compiler, but also high-level language code that is executable by a computer by using an interpreter or the like.

The term 'the' and other demonstratives similar thereto in the specification of the present disclosure (especially in the following claims) should be understood to include a singular form and plural forms. Furthermore, recitation of ranges of values herein are merely intended to serve as a shorthand method of referring individually to each separate value falling within the range, unless otherwise indicated herein, and each separate value is incorporated into the specification as if it were individually recited herein.

The operations of the methods according to the present disclosure may be performed in any suitable order unless otherwise indicated herein or otherwise clearly contradicted by context. The present disclosure is not limited to the described order of the operations. The use of any and all examples, or exemplary language (e.g., 'and the like') provided herein, is intended merely to better illuminate the present disclosure and does not pose a limitation on the scope of the present disclosure unless otherwise claimed. Also, numerous modifications and adaptations will be readily apparent to those skilled in the art without departing from the scope of the present disclosure.

Accordingly, the present disclosure should not be limited to the above-described embodiments, and all modifications and variations which may be derived from the meanings, scopes and equivalents of the claims should be construed as falling within the scope of the present disclosure.

## Claims

1. A method, performed by a processor (180) of an Alzheimer's disease diagnosis apparatus (100), of diagnosing Alzheimer's disease, the method comprising:
extracting features of speech data stored in a database, the speech data being speech data of a speaker; and
generating, based on the features of the speech data, at least one of an Alzheimer's disease classification result and a cognitive function assessment score prediction result
wherein the generating comprises:
generating a first Alzheimer's disease classification result and a first cognitive function assessment score prediction result, based on a result of selecting features of a speech section of first speech data uttered by the speaker in response to a first speech task that requests the speaker's response to one or more preset questions;
generating a second Alzheimer's disease classification result and a second cognitive function assessment score prediction result, based on a result of selecting features of a speech section of second speech data uttered by the speaker in response to a second speech task that outputs an audio narration of a preset story and requests the speaker to repeat the audio narration; and
generating a third Alzheimer's disease classification result and a third cognitive function assessment score prediction result, based on a result of selecting features of a speech section of third speech data uttered by the speaker in response to a third speech task that requests a recall of a story.

2. The method of claim 1, further comprising, before the extracting of the features of the speech data, separating the speech data of the speaker into a speech section and a pause section,
wherein the extracting of the features of the speech data comprises extracting, from the speech data included in the speech section, at least one of a frequency-related feature, a loudness-related feature, a temporal feature, and a spectrum feature.

3. The method of claim 1, further comprising, after the extracting of the features of the speech data, selecting features of the speech data to be used for generating the Alzheimer's disease classification result and the cognitive function assessment score prediction result.

4. The method of claim 3, wherein the selecting of the features of the speech data comprises:
loading the features of the speech data that are included in a speech section separated from the speech data of the speaker;
analyzing correlations between the features of the speech data and a presence or absence of Alzheimer's disease through repetitive measurements of variance analysis, by using the features of the speech data as independent variables and the presence or absence of Alzheimer's disease as a dependent variable; and
selecting the features of the speech data that are below a preset significance level, from a result of the analyzing the correlations.

5. The method of claim 4, wherein the generating comprises:
generating the Alzheimer's disease classification result corresponding to the features of the speech data that are below the preset significance level, by using a first deep neural network model that is pre-trained to classify a presence or absence of Alzheimer's disease in response to the features of the speech data; and
generating the cognitive function assessment score prediction result corresponding to the features of the speech data that are below the preset significance level, by using a second deep neural network model that is pre-trained to predict a cognitive function assessment score in response to the features of the speech data,
the first deep neural network model is a model trained in a supervised learning manner with training data comprising features of speech data as inputs and a presence or absence of Alzheimer's disease as a label, and
the second deep neural network model is a model trained in a supervised learning manner with training data comprising features of speech data as inputs and a cognitive function assessment score as a label.

6. The method of claim 5**,** wherein the generating comprises simultaneously generating the Alzheimer's disease classification result and the cognitive function assessment score prediction result corresponding to the features of the speech data that are below the preset significance level.

7. The method of claim 1, wherein the generating comprises:
generating the Alzheimer's disease classification result based on results of selecting features of speech sections of the first speech data and the second speech data;
determining, based on the Alzheimer's disease classification result, whether to execute generation of the cognitive function assessment score prediction result; and
based on determining to execute the generation of the cognitive function assessment score prediction result, generating the cognitive function assessment score prediction result based on a result of selecting features of a speech section of the third speech data, excluding the features of the first speech data and the features of the second speech data.

8. The method of claim 1, wherein the determining comprises, based on the Alzheimer's disease classification result for at least one of the features of the first speech data and the second speech data being generated as a first value, determining to execute the generation of the cognitive function assessment score prediction result.

9. A computer-readable recording medium having stored therein a computer program for causing a computer to execute the method of any one of claims 1 to 8.

10. An Alzheimer's disease diagnosis apparatus (100) comprising:
a processor (180); and
a memory (190) operably connected to the processor (180) and storing at least one piece of code to be executed by the processor (180),
wherein the memory stores code that, when executed by the processor (180), causes the processor (180) to
extract features of speech data of a speaker, and
generate, based on the features of the speech data, at least one of an Alzheimer's disease classification result and a cognitive function assessment score prediction result, wherein generating the at least one of an Alzheimer's disease classification result and a cognitive function assessment score prediction result comprises:
generate a first Alzheimer's disease classification result and a first cognitive function assessment score prediction result, based on a result of selecting features of a speech section of first speech data uttered by the speaker in response to a first speech task that requests the speaker's response to one or more preset questions,
generate a second Alzheimer's disease classification result and a second cognitive function assessment score prediction result, based on a result of selecting features of a speech section of second speech data uttered by the speaker in response to a second speech task that outputs an audio narration of a preset story and requests the speaker to repeat the audio narration and
generate a third Alzheimer's disease classification result and a third cognitive function assessment score prediction result, based on a result of selecting features of a speech section of third speech data uttered by the speaker in response to a third speech task that requests a recall of a story.

## Patentansprüche

1. Verfahren, das durch den Prozessor (180) einer Morbus-Alzheimer-Diagnosevorrichtung (100) durchgeführt wird, wobei das Verfahren Folgendes umfasst:
Extrahieren von Merkmalen von Sprachdaten, die in einer Datenbank gespeichert sind, wobei die Sprachdaten Sprachdaten eines Sprechers sind; und
Erzeugen, basierend auf den Merkmalen der Sprachdaten, mindestens eines von einem Morbus-Alzheimer-Klassifikationsergebnis und einem Punktzahlvorhersageergebnis für einen kognitiven Funktionstest,
wobei das Erzeugen Folgendes umfasst:
Erzeugen eines ersten Morbus-Alzheimer-Klassifikationsergebnisses und eines ersten Bewertungspunktzahlvorhersageergebnisses für einen kognitiven Funktionstest basierend auf einem Ergebnis eines Auswählens von Merkmalen eines Sprachabschnitts erster Sprachdaten, die durch den Sprecher als Reaktion auf eine erste Sprachaufgabe, die eine Antwort des Sprechers auf eine oder mehrere voreingestellte Fragen erbittet, geäußert werden;
Erzeugen eines zweiten Morbus-Alzheimer-Klassifikationsergebnisses und eines zweiten Bewertungspunktzahlvorhersageergebnisses für einen kognitiven Funktionstest basierend auf einem Ergebnis eines Auswählens von Merkmalen eines Sprachabschnitts zweiter Sprachdaten, die durch den Sprecher als Reaktion auf eine zweite Sprachaufgabe, die eine Audioerzählung einer voreingestellten Geschichte ausgibt und den Sprecher bittet, die Audioerzählung zu wiederholen, geäußert werden; und
Erzeugen eines dritten Morbus-Alzheimer-Klassifikationsergebnisses und eines dritten Bewertungspunktzahlvorhersageergebnisses für einen kognitiven Funktionstest basierend auf einem Ergebnis eines Auswählens von Merkmalen eines Sprachabschnitts dritter Sprachdaten, die durch den Sprecher als Reaktion auf eine dritte Sprachaufgabe, die eine Erinnerung an eine Geschichte erbittet, geäußert werden.

2. Verfahren nach Anspruch 1, ferner umfassend, vor dem Extrahieren der Merkmale der Sprachdaten, Trennen der Sprachdaten des Sprechers in einen Sprachabschnitt und einen Pausenabschnitt,
wobei das Extrahieren der Merkmale der Sprachdaten Extrahieren, aus den Sprachdaten, die in dem Sprachabschnitt beinhaltet sind, mindestens eines von einem häufigkeitsbezogenen Merkmal, einem lautstärkebezogenen Merkmal, einem temporalen Merkmal und einem Spektrumsmerkmal umfasst.

3. Verfahren nach Anspruch 1, ferner umfassend, nach dem Extrahieren der Merkmale der Sprachdaten, Auswählen von Merkmalen der Sprachdaten, die zum Erzeugen des Morbus-Alzheimer-Klassifikationsergebnisses und des Punktzahlvorhersageergebnisses für einen kognitiven Funktionstest zu verwenden sind.

4. Verfahren nach Anspruch 3, wobei das Auswählen der Merkmale der Sprachdaten Folgendes umfasst:
Laden der Merkmale der Sprachdaten, die in einem Sprachabschnitt, der von den Sprachdaten des Sprechers getrennt ist, beinhaltet sind;
Analysieren von Korrelationen zwischen den Merkmalen der Sprachdaten und einem Vorhandensein oder einer Abwesenheit von Morbus Alzheimer durch repetitive Messungen von Varianzanalysen unter Verwendung der Merkmale der Sprachdaten als unabhängige Variablen und des Vorhandenseins oder der Abwesenheit von Morbus Alzheimer als einer abhängigen Variablen; und
Auswählen der Merkmale der Sprachdaten, die unterhalb eines voreingestellten Signifikanzniveaus liegen, aus einem Ergebnis der Analyse der Korrelationen.

5. Verfahren nach Anspruch 4, wobei das Erzeugen Folgendes umfasst:
Erzeugen des Morbus-Alzheimer-Klassifikationsergebnisses, das den Merkmalen der Sprachdaten entspricht, die unterhalb des voreingestellten Signifikanzniveaus liegen, unter Verwendung eines ersten tiefen neuronalen Netzwerkmodells, das vortrainiert ist, ein Vorhandensein oder eine Abwesenheit von Morbus Alzheimer als Reaktion auf die Merkmale der Sprachdaten zu klassifizieren; und
Erzeugen des Punktzahlvorhersageergebnisses für einen kognitiven Funktionstest, das den Merkmalen der Sprachdaten entspricht, die unterhalb des voreingestellten Signifikanzniveaus liegen, unter Verwendung eines zweiten tiefen neuronalen Netzwerkmodells, das vortrainiert ist, eine Punktzahl für einen kognitiven Funktionstest als Reaktion auf die Merkmale der Sprachdaten vorherzusagen,
wobei das erste tiefe neuronale Netzwerkmodell ein Modell ist, das in einer überwachten Lernweise mit Trainingsdaten trainiert wurde, die Merkmale von Sprachdaten als Eingaben und ein Vorhandensein oder eine Abwesenheit von Morbus Alzheimer als eine Kennung umfassen, und
wobei das zweite tiefe neuronale Netzwerkmodell ein Modell ist, das in einer überwachten Lernweise mit Trainingsdaten trainiert wurde, die Merkmale von Sprachdaten als Eingaben und eine Punktzahl eines kognitiven Funktionstests als eine Kennung umfassen.

6. Verfahren nach Anspruch 5, wobei das Erzeugen gleichzeitiges Erzeugen des Morbus-Alzheimer-Klassifikationsergebnisses und des Punktzahlvorhersageergebnisses für einen kognitiven Funktionstest, das den Merkmalen der Sprachdaten entspricht, die unterhalb des voreingestellten Signifikanzniveaus liegen, umfasst.

7. Verfahren nach Anspruch 1, wobei das Erzeugen Folgendes umfasst:
Erzeugen des Morbus-Alzheimer-Klassifikationsergebnisses basierend auf Ergebnissen des Auswählens von Merkmalen von Sprachabschnitten der ersten Sprachdaten und der zweiten Sprachdaten;
Bestimmen, basierend auf dem Morbus-Alzheimer-Klassifikationsergebnis, ob die Erzeugung des Punktzahlvorhersageergebnisses für einen kognitiven Funktionstest auszuführen ist; und
basierend auf dem Bestimmen, die Erzeugung des Punktzahlvorhersageergebnisses für einen kognitiven Funktionstest auszuführen, Erzeugen des Punktzahlvorhersageergebnisses für einen kognitiven Funktionstest basierend auf einem Ergebnis des Auswählens von Merkmalen eines Sprachabschnitts der dritten Sprachdaten, unter Ausschluss der Merkmale der ersten Sprachdaten und der Merkmale der zweiten Sprachdaten.

8. Verfahren nach Anspruch 1, wobei das Bestimmen, basierend darauf, dass das Morbus-Alzheimer-Klassifikationsergebnis für mindestens eines der Merkmale der ersten Sprachdaten und der zweiten Sprachdaten als ein erster Wert erzeugt wird, Bestimmen, die Erzeugung des Punktzahlvorhersageergebnisses für einen kognitiven Funktionstest auszuführen, umfasst.

9. Computerlesbares Aufzeichnungsmedium, das ein darauf gespeichertes Computerprogramm zum Veranlassen eines Computers, das Verfahren nach einem der Ansprüche 1 bis 8 auszuführen, aufweist.

10. Morbus-Alzheimer-Diagnosevorrichtung (100), umfassend:
einen Prozessor (180); und
einen Speicher (190), der mit dem Prozessor (180) wirkverbunden ist und mindestens ein Stück Code speichert, der durch den Prozessor (180) auszuführen ist,
wobei der Speicher Code speichert, der, wenn er durch den Prozessor (180) ausgeführt wird, den Prozessor (180) dazu veranlasst, Merkmale von Sprachdaten eines Sprechers zu extrahieren, und
Erzeugen, basierend auf den Merkmalen der Sprachdaten, mindestens eines von einem Morbus-Alzheimer-Klassifikationsergebnis und einem Punktzahlvorhersageergebnis für einen kognitiven Funktionstest, wobei das Erzeugen des mindestens einen von einem Morbus-Alzheimer-Klassifikationsergebnis und einem Punktzahlvorhersageergebnis für einen kognitiven Funktionstest Folgendes umfasst:
Erzeugen eines ersten Morbus-Alzheimer-Klassifikationsergebnisses und eines ersten Bewertungspunktzahlvorhersageergebnisses für einen kognitiven Funktionstest basierend auf einem Ergebnis eines Auswählens von Merkmalen eines Sprachabschnitts erster Sprachdaten, die durch den Sprecher als Reaktion auf eine erste Sprachaufgabe, die eine Antwort des Sprechers auf eine oder mehrere voreingestellte Fragen erbittet, geäußert werden,
Erzeugen eines zweiten Morbus-Alzheimer-Klassifikationsergebnisses und eines zweiten Bewertungspunktzahlvorhersageergebnisses für einen kognitiven Funktionstest basierend auf einem Ergebnis eines Auswählens von Merkmalen eines Sprachabschnitts zweiter Sprachdaten, die durch den Sprecher als Reaktion auf eine zweite Sprachaufgabe, die eine Audioerzählung einer voreingestellten Geschichte ausgibt und den Sprecher bittet, die Audioerzählung zu wiederholen, geäußert werden, und
Erzeugen eines dritten Morbus-Alzheimer-Klassifikationsergebnisses und eines dritten Bewertungspunktzahlvorhersageergebnisses für einen kognitiven Funktionstest basierend auf einem Ergebnis eines Auswählens von Merkmalen eines Sprachabschnitts dritter Sprachdaten, die durch den Sprecher als Reaktion auf eine dritte Sprachaufgabe, die eine Erinnerung an eine Geschichte erbittet, geäußert werden.

## Revendications

1. Procédé de diagnostic de la maladie d'Alzheimer, réalisé par un processeur (180) d'un appareil de diagnostic de la maladie d'Alzheimer (100), le procédé comprenant :
l'extraction de caractéristiques de données de parole stockées dans une base de données, les données de parole étant des données de parole d'un locuteur ; et
la génération, à partir des caractéristiques des données de parole, d'au moins un résultat parmi un résultat de classification de la maladie d'Alzheimer et un résultat de prédiction de score d'évaluation de fonction cognitive,
dans lequel la génération comprend :
la génération d'un premier résultat de classification de la maladie d'Alzheimer et d'un premier résultat de prédiction de score d'évaluation de fonction cognitive, à partir d'un résultat de sélection de caractéristiques d'une section de parole de premières données de parole prononcées par le locuteur en réponse à une première tâche de parole qui requiert la réponse du locuteur à une ou plusieurs questions préréglées ;
la génération d'un deuxième résultat de classification de la maladie d'Alzheimer et d'un deuxième résultat de prédiction de score d'évaluation de fonction cognitive, à partir d'un résultat de sélection de caractéristiques d'une section de parole de deuxièmes données de parole prononcées par le locuteur en réponse à une deuxième tâche de parole qui émet une narration audio d'une histoire préréglée et requiert que le locuteur répète la narration audio ; et
la génération d'un troisième résultat de classification de la maladie d'Alzheimer et d'un troisième résultat de prédiction de score d'évaluation de fonction cognitive, à partir d'un résultat de sélection de caractéristiques d'une section de parole de troisièmes données de parole prononcées par le locuteur en réponse à une troisième tâche de parole qui requiert un rappel d'une histoire.

2. Procédé selon la revendication 1, comprenant en outre, avant l'extraction des caractéristiques des données de parole, la séparation des données de parole du locuteur en une section de parole et une section de pause,
dans lequel l'extraction des caractéristiques des données de parole comprend l'extraction, à partir des données de parole incluses dans la section de parole, d'au moins une caractéristique parmi une caractéristique liée à la fréquence, une caractéristique liée à l'intensité sonore, une caractéristique temporelle et une caractéristique de spectre.

3. Procédé selon la revendication 1, comprenant en outre, après l'extraction des caractéristiques des données de parole, la sélection de caractéristiques des données de parole à utiliser pour générer le résultat de classification de la maladie d'Alzheimer et le résultat de prédiction de score d'évaluation de fonction cognitive.

4. Procédé selon la revendication 3, dans lequel la sélection des caractéristiques des données de parole comprend :
le chargement des caractéristiques des données de parole qui sont incluses dans une section de parole séparée provenant des données de parole du locuteur ;
l'analyse de corrélations entre les caractéristiques des données de parole et une présence ou absence de la maladie d'Alzheimer par des mesures répétitives d'analyse de variance, en utilisant les caractéristiques des données de parole comme variables indépendantes et la présence ou absence de la maladie d'Alzheimer comme une variable dépendante ; et
la sélection des caractéristiques des données de parole qui sont inférieures à un niveau de signification préréglé, à partir d'un résultat de l'analyse des corrélations.

5. Procédé selon la revendication 4, dans lequel la génération comprend :
la génération du résultat de classification de la maladie d'Alzheimer correspondant aux caractéristiques des données de parole qui sont inférieures au niveau de signification préréglé, en utilisant un premier modèle de réseau de neurones profonds qui est pré-entraîné pour classifier une présence ou absence de la maladie d'Alzheimer en réponse aux caractéristiques des données de parole ; et
la génération du résultat de prédiction de score d'évaluation de fonction cognitive correspondant aux caractéristiques des données de parole qui sont inférieures au niveau de signification préréglé, en utilisant un second modèle de réseau de neurones profonds qui est pré-entraîné pour prédire un score d'évaluation de fonction cognitive en réponse aux caractéristiques des données de parole,
le premier modèle de réseau de neurones profonds est un modèle entraîné d'une manière d'apprentissage supervisé avec des données d'entraînement comprenant des caractéristiques de données de parole comme entrées et une présence ou absence de la maladie d'Alzheimer comme une étiquette, et
le second modèle de réseau de neurones profonds est un modèle entraîné d'une manière d'apprentissage supervisé avec des données d'entraînement comprenant des caractéristiques de données de parole comme entrées et un score d'évaluation de fonction cognitive comme une étiquette.

6. Procédé selon la revendication 5, dans lequel la génération comprend la génération simultanée du résultat de classification de la maladie d'Alzheimer et du résultat de prédiction de score d'évaluation de fonction cognitive correspondant aux caractéristiques des données de parole qui sont inférieures au niveau de signification préréglé.

7. Procédé selon la revendication 1, dans lequel la génération comprend :
la génération du résultat de classification de la maladie d'Alzheimer à partir de résultats de sélection de caractéristiques de sections de parole des premières données de parole et des deuxièmes données de parole ;
la détermination, à partir du résultat de classification de la maladie d'Alzheimer, s'il faut exécuter une génération du résultat de prédiction de score d'évaluation de fonction cognitive ; et
à partir de la détermination d'exécuter la génération du résultat de prédiction de score d'évaluation de fonction cognitive, la génération du résultat de prédiction de score d'évaluation de fonction cognitive à partir d'un résultat de sélection de caractéristiques d'une section de parole des troisièmes données de parole, à l'exclusion des caractéristiques des premières données de parole et des caractéristiques des deuxièmes données de parole.

8. Procédé selon la revendication 1, dans lequel la détermination comprend, à partir du fait que le résultat de classification de la maladie d'Alzheimer pour au moins une des caractéristiques des premières données de parole et des deuxièmes données de parole est généré comme une première valeur, la détermination d'exécuter la génération du résultat de prédiction de score d'évaluation de fonction cognitive.

9. Support d'enregistrement lisible par ordinateur ayant stocké dans celui-ci un programme informatique pour amener un ordinateur à réaliser le procédé selon l'une quelconque des revendications 1 à **8.**

10. Appareil de diagnostic de la maladie d'Alzheimer (100) comprenant :
un processeur (180) ; et
une mémoire (190) connectée de manière opérationnelle au processeur (180) et stockant au moins un élément de code à réaliser par le processeur (180),
dans lequel la mémoire stocke du code qui, lorsqu'il est réalisé par le processeur (180), amène le processeur (180) à extraire des caractéristiques de données de parole d'un locuteur, et
générer, à partir des caractéristiques des données de parole, au moins un résultat parmi un résultat de classification de la maladie d'Alzheimer et un résultat de prédiction de score d'évaluation de fonction cognitive, dans lequel la génération de l'au moins un résultat parmi un résultat de classification de la maladie d'Alzheimer et un résultat de prédiction de score d'évaluation de fonction cognitive comprend :
la génération d'un premier résultat de classification de la maladie d'Alzheimer et d'un premier résultat de prédiction de score d'évaluation de fonction cognitive, à partir d'un résultat de sélection de caractéristiques d'une section de parole de premières données de parole prononcées par le locuteur en réponse à une première tâche de parole qui requiert la réponse du locuteur à une ou plusieurs questions préréglées,
la génération d'un deuxième résultat de classification de la maladie d'Alzheimer et d'un deuxième résultat de prédiction de score d'évaluation de fonction cognitive, à partir d'un résultat de sélection de caractéristiques d'une section de parole de deuxièmes données de parole prononcées par le locuteur en réponse à une deuxième tâche de parole qui émet une narration audio d'une histoire préréglée et requiert que le locuteur répète la narration audio, et
la génération d'un troisième résultat de classification de la maladie d'Alzheimer et d'un troisième résultat de prédiction de score d'évaluation de fonction cognitive, à partir d'un résultat de sélection de caractéristiques d'une section de parole de troisièmes données de parole prononcées par le locuteur en réponse à une troisième tâche de parole qui requiert un rappel d'une histoire.
